(19) Europäisches Patentamt
European Patent Office
Office européen des brevets

(11) Publication number: 0 207 696
A1

(12) # EUROPEAN PATENT APPLICATION

(21) Application number: 86304754.4

(22) Date of filing: 20.06.86

(51) Int. Cl.⁴: C 07 D 277/82
C 07 D 263/58, A 61 K 31/425
A 61 K 31/42

(30) Priority: 24.06.85 US 747748

(43) Date of publication of application:
07.01.87 Bulletin 87/2

(84) Designated Contracting States:
AT BE CH DE FR GB IT LI NL SE

(71) Applicant: ELI LILLY AND COMPANY
Lilly Corporate Center
Indianapolis Indiana 46285(US)

(72) Inventor: Laguzza, Bennett Coleman
7340 North Grand Avenue
Indianapolis Indiana, 46250(US)

(72) Inventor: TUrner, William Wilson, Jr.
321 East 14th Street Apt. A3
Bloomington Indiana 47401(US)

(74) Representative: Tapping, Kenneth George et al,
Erl Wood Manor
Windlesham Surrey, GU20 6PH(GB)

(54) Dialkylaminotetrahydrobenzothiazoles and oxazoles.

(57) ±-2-Amino-6-dialkylaminotetrahydrobenzothiazoles or oxazoles as receptor agonists.

EP 0 207 696 A1

Croydon Printing Company Ltd

# Dialkylaminotetrahydrobenzothiazoles and Oxazoles

Certain complex amides of lysergic acid (I, R=OH--9-ergolene-8β-carboxylic acid) are found in ergoted rye; ie, rye contaminated by the growth of the filamentous fungus Claviceps purpurea. Persons eating bread prepared from ergoted rye were subject to ergot poisoning, known in the Middle Ages as St. Anthony's Fire because of the intense feeling of heat in the extremities. Ergot poisoning was often fatal. The peripheral vasoconstrictor properties of the ergot alkaloids as a group are responsible for the fatalities seen with ergot poisoning, with gangrene of the extremeties being a common precipitating factor.

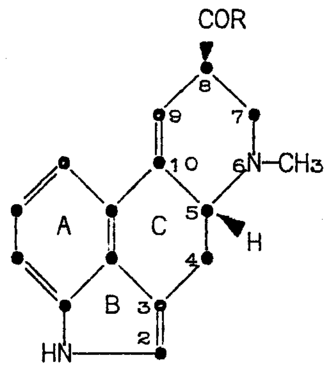

I

Two dozen ergot alkaloids have been characterized from isolates from Claviceps purpurea infestations. Derivatives of lysergic acid include the simple amides, ergine

($R=NH_2$) and ergonovine (ergometrine) (I, $-R=N-\overset{CH_3}{\overset{|}{C}}HCH_2OH$), as well as the peptide alkaloids (R=a complex amide derived from a polypeptide by cyclization) including ergotamine, ergosine, ergocornine, ergocryptine, ergocristine, etc. The corresponding alkaloids based on isolysergic acid (9-ergolene-8α-carboxylic acid) have also been isolated.

The ergot alkaloids as a group have several interesting pharmacologic activities; uterine contraction (oxytocic action), peripheral vasoconstriction, adrenergic blockade, and serotonin antagonism plus varied CNS activities including the production of hallucinations. Certain of the alkaloids individually are used to produce post-partum uterine contractions and in the sympomatic treatment of migraine. Pharmacologic activity, toxicity and central effects vary from alkaloid to alkaloid. In general, hydrogenation of the delta-9 double bond results in compounds of lowered activity as regards peripheral action but adrenolytic and central inhibition of vasomotor centers may be enhanced.

Derivatives of lysergic acid and dihydrolysergic acid are too numerous to mention, but include, generically, substitution on the indole nitrogen, at C-2 (α-bromocryptine is a 2-bromo derivative), replacement of N-6 methyl by other alkyl groups or by allyl and replacement of the carboxamide function at C-8 by various groups, particularly cyanomethyl, carboxamidomethyl, methylthiomethyl, and methoxymethyl. In

addition, there has been substitution of simpler amide groups (butanolamide=methyl ergonovine diethyl amide) for the complex "polypeptide chains" of the natural alkaloids.

In the past, there has been considerable speculation, frequently followed by a synthetic effort, as to what portions of the ergoline molecule are responsible for pharmacologic activity; ie, do part-structures exist which retain, perhaps selectively, certain pharmacologic activities of the parent alkaloids? One part-structure which has been thoroughly examined is the amino tetrahydronaphthalene structure II from which the B and D rings of an ergoline have been subtracted.

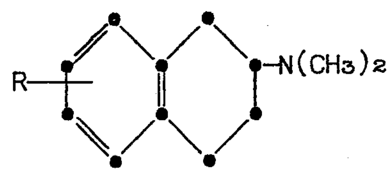

II

where R is carboxamide, hydroxy, amino etc. The elements of a β-phenethylamine can also be discerned from I using the phenyl ring and carbons 5 and 10 plus the nitrogen at 6, a part structures in which only the A ring is retained. In addition, tricyclics lacking the B (pyrrole) ring as well as benz[cd]indoles (lacking the D ring - see United States patent 4,110,339) have been prepared. None of these part-structures seemed to have the desired

degree of specificity as regards the dopamine D-2 agonist activity (prolactin inhibition etc) of ergocornine, dihydroergocornine, lergotrile or pergolide (United States patents 3,920,664, 4,054,660 and 4,166,182 for example). Recently, however, Kornfeld and Bach have found that the A ring of an ergoline is not required for D-2 agonist activity. These new part structures are named as hexahydropyrrolo[4,3-g]quinolines- see United States patent 4,235,909. A related structure, a hexahydropyrazolo[4,3-g]quinoline, United States patent 4,198,415, also has excellent D-2 agonist action. The corresponding 2-ring compounds, in which the D ring of the ergoline is opened, are also active D-2 agonists -- see United States patent 4,235,226 for the amino-substituted isoindoles, and United States patent 4,276,300 for the amino substituted indazoles (like the three-ring pyrazoles). It has now been found that other hetero ring systems than pyrrole and pyrazole can be attached to the perhydroquinoline ring (rings B + C of an ergoline) to complete a three-ring analogue. One of these is a pyrimidine - see Nichols and Kornfeld, United States patent 4,501,890.

2-Amino-6-dialkylaminotetrahydrobenzothiozoles and oxazoles are not known.

This invention provides tetrahydrobenzothiazoles and oxazoles of the formula

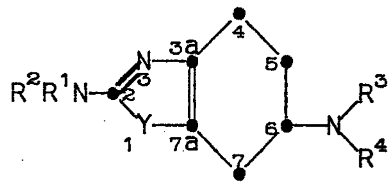

XX

wherein Y is S or O, $R^1$ and $R^2$ are independently H, methyl, ethyl or n-propyl, and $R^3$ and $R^4$ are independently H, methyl, ethyl, n-propyl or allyl; and pharmaceutically-acceptable acid addition salts thereof formed with non-toxic acids.

Compounds wherein Y is S are substituted-4,5,6,7-tetrahydrobenzothiazoles and those wherein Y is O are substituted-4,5,6,7-tetrahydrobenzoxazoles.

Compounds according to XX wherein one or both of $R^3$ and $R^4$ is H are intermediates in that they can be allylated or alkylated to yield compounds of this invention wherein $R^3$ and $R^4$ are individually methyl, ethyl, n-propyl or allyl.

A preferred group of compounds according to XX are those in which $R^3$ and $R^4$ are both methyl, both n-propyl or both allyl. Another preferred group are those compounds according to XX in which $R^1$ and $R^2$ are both H.

The compounds of this invention wherein neither $R^3$ nor $R^4$ is H have receptor agonist activity; ie, they can increase the amounts centrally or peripherally or both of certain neurohormones. For example, compounds according to XX in which both $R^3$ and $R^4$ are allyl or n-propyl, are dopamine agonists, particularly dopamine D-2 agonists, capable of increasing the effective concentration of dopamine in the brain. Compounds wherein $R^3$ and $R^4$ are both methyl have α-agonist action.

Also included within the scope of this invention are acid addition salts of the nitrogenous bases represented by XX. The above compounds contain at least three basic nitrogens; first, the amine group at C-6; secondly, the ring nitrogen in the fused heterocyclic ring; and thirdly, the $NR^1R^2$ group. The nitrogen of the C-6 amino group is generally the most basic of the amine functions and readily forms acid addition salts. Strong inorganic acids such as the mineral acids or strong organic acids, such as p-toluenesulfonic acid, can when employed in excess form di salts with one of the other amine functions in the compounds of this invention.

Pharmaceutically-acceptable acid addition salts of the compounds of this invention thus include mono or di salts derived from inorganic acids such as: hydrochloric acid, nitric acid, phosphoric acid, sulfuric acid, hydrobromic acid, hydriodic acid, phosphorous acid and the like, as well as salts derived from organic acids such as aliphatic mono and dicarboxylic acids, phenyl-substituted alkanoic acids, hydroxyalkanoic and alkandioic acids, aromatic acids, aliphatic and aromatic sulfonic acids, etc. Such pharmaceutically-acceptable salts thus include sulfate, pyrosulfate, bisulfate, sulfite, bisulfite, nitrate, phosphate,

monohydrogenphosphate, dihydrogenphosphate, metaphosphate, pyrophosphate, chloride, bromide, iodide, fluoride, acetate, propionate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, α-hydroxybutyrate, glycollate, malate, tartrate, methanesulfonate, propanesulfonate, naphthalene-1-sulfonate, naphthalene-2-sulfonate and the like salts.

The compounds of this invention according to XX above, where $R^3$ and $R^4$ are individually only methyl, ethyl or n-propyl ($R^5$ and $R^6$) can be prepared according to the following reaction scheme:

Reaction Scheme I

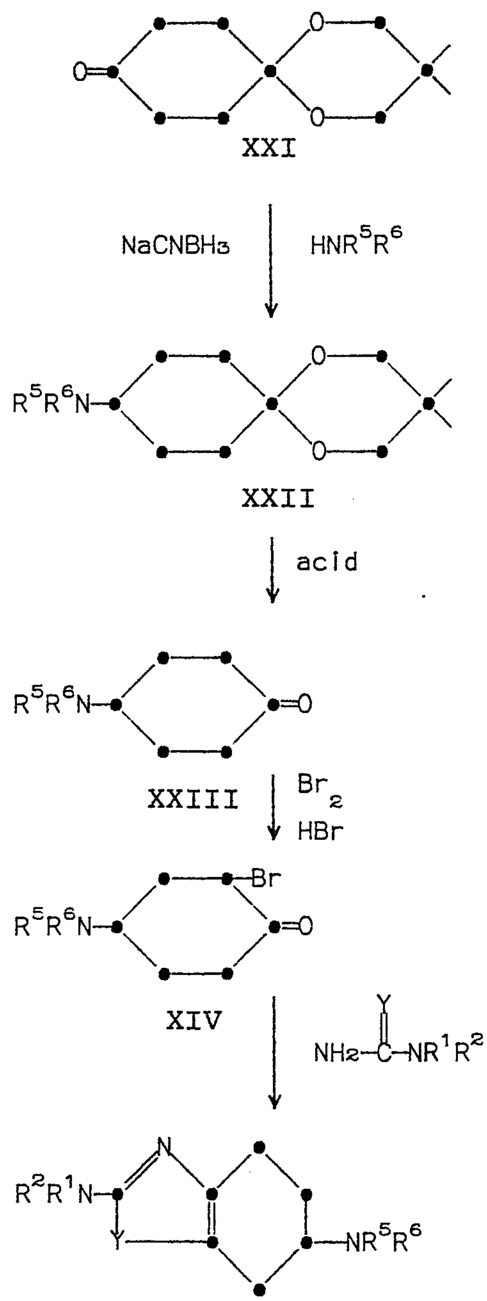


XXa

wherein Y, $R^1$ and $R^2$ have their previous meanings and $R^5$ and $R^6$ are individually methyl, ethyl or n-propyl.

In Reaction Scheme I above, a protected, as by ketal formation, cyclohexan-1,4-dione, is reductively aminated with a secondary amine, $HNR^5R^6$, using a borohydride in a mutual inert solvent as the reducing agent. As a starting material, the ketal of cyclohexane-1,4-dione with 2,2-dimethyl-1,3-propanediol (XXI) is commercially available and is therefore preferred although monoketals with other diols, ie, ethylenediol, 1,3-propylenediol and the like, may also be used. Sodium cyanoborohydride is the reducing agent of choice, although other metal hydrides can also be employed. Ethers, and particularly cyclic ethers such as THF, are the solvents of choice for this reaction.

The product of the reductive amination, XXII, is next deprotected by treatment with acid to yield a 4-dialkylaminocyclohexanone. Bromination alpha to the carbonyl with bromine in HBr/acetic acid yields the alpha-bromoketone, XIV. Reaction of the bromoketone with urea, thiourea, an N-alkyl urea, an N,N-dialkyl urea, an N-alkyl or an N,N-dialkyl thiourea yields directly XXa.

Compounds according to XX wherein one or both of $R^3$ and $R^4$ are allyl are prepared by dealkylating a dialkyl amine XX where $R^3$ and $R^4$ are methyl (or XXa where $R^5$ and $R^6$ are methyl) with CNBr to yield an intermediate compound wherein one or both $R^3$ and $R^4$ (or $R^5$ and $R^6$) are H and then reductively allylating this amine with acrylic acid or directly allylating with

allylbromide in the presence of a base. If desired, a primary amine group at C-2 can be protected as by acylation, and the acyl group removed by hydrolysis after the allylation at the C-6 amine is completed.

Compounds according to XX above have an asymmetric carbon, the C-6 carbon, to which the amino group ($NR^3R^4$) is attached. Thus, compounds represented by XX above include two optical isomers occurring as a (±) or dl pair or racemate. Resolution of a (±) pair of this invention into its optical antipodes can be accomplished by the usual empirical procedures used by those skilled in the art. Such separated optical isomers are included within the scope of this invention.

Compounds preparable by the above procedures which illustrate the scope of this invention include:

(-)-2,6-bis(dimethylamino)-5,6,7,8-tetrahydrobenzothiazole methanesulfonate,

(+)-2,6-bis(methyl-n-propylamino)-5,6,7,8-tetrahydrobenzoxazole hydrobromide,

(±)-2-dimethylamino-6-ethyl-n-propylamino-5,6,7,8-tetrahydrobenzoxazole sulfate,

(±)-2-diethylamino-6-di-n-propylamino-5,6,7,8-tetrahydrobenzothiazole hydrobromide,

(±)-2-methylethylamino-6-diallylamino-5,6,7,8-tetrahydrobenzothiazole maleate,

(-)-2-dimethylamino-6-allylmethylamino-5,6,7,8-tetrahydrobenzoxazole succinate,

(±)-2-diethylamino-6-allyl-n-propyl-5,6,7,8-tetrahydrobenzothiazole tartrate,

(-)-2-amino-6-diallylamino-5,6,7,8-tetrahydrobenzothiazole dinitrobenzoate,

(±)-2-ethylmethylamine-6-dimethylamino-5,6,7,8-tetrahydrobenzoxazole phosphate and the like.

This invention is further illustrated by the following specific examples.

## Example 1

Preparation of (±)-2-Amino-6-di-n-propylamino-5,6,7,8-tetrahydrobenzothiazole

About 125 ml of methanol were added to a 500 ml round bottom flask under a nitrogen blanket. Forty-two ml of di-n-propylamine were added and the resulting solution cooled to about 0° in an ice/water bath. Twenty ml of 5N methanolic hydrochloric acid were added. The reaction mixture was stirred until salt formation was complete. Ten grams of 1,4-cyclohexanedione-mono-2,2-dimethyltrimethylene ketal were added as a solid. The reaction mixture was stirred with cooling for several minutes at which point 2.25 g of sodium cyanoborohydride were added in one portion. A white slurry developed rapidly which was stirred in the cold for several minutes and then at room temperature under a nitrogen blanket for about 30 hours. An additional 2.2 g of sodium cyanoborohydride were added. After an additional 24 hours stirring at room temperature, at which time TLC (chloroform/methanol 95:5) indicated a new spot and the absence of a spot corresponding to

starting material. The reaction mixture was therefore filtered and the filter cake washed with methanol. The filtrate and wash were combined, and the solvent evaporated therefrom to yield a thick, white slurry. The slurry was cooled to about 0°C and 100 ml of 1N hydrochloric acid were added with stirring. The resulting solution was extracted twice with equal volumes of ether. The ether extracts were separated and discarded. The aqueous layer was cooled and then made basic by the addition of 5N aqueous sodium hydroxide (pH ~12). The basic layer was extracted three times with 50 ml portions of methylene dichloride. The methylene dichloride extracts were combined, the combined extracts were washed once with saturated aqueous sodium bicarbonate and were then dried. Evaporation of the solvent *in vacuo* yielded a yellow oil containing some solid; weight = 6.26 g. The residue was flash chromatographed over silica using a 100:1 ether/triethylamine solvent as eluant. [The term "flash chromatography" refers to a procedure developed by Still *et al.*, *J. Org. Chem.*, *43*, 2923 (1978)]. Fifty ml fraction were taken. Fractions 10-15 were shown by TLC to contain the desired material. These fractions were combined and concentrated *in vacuo* to yield 5.40 g of a nearly colorless oil comprising 4-di-n-propylamino-1-cyclohexanone 2,2-dimethyltrimethylene ketal.

The ketal group was removed by dissolving 4.4 g of the above ketal in 110 ml of 6N hydrochloric acid with stirring under a nitrogen atmosphere for 48 hours at room temperature. The colorless solution

was extracted with 50 ml of ether; the ether extract was discarded. The acidic solution was then made basic by the addition of an excess of 5N aqueous sodium hydroxide. The resulting cloudy mixture was extracted three times with 50 ml portions of methylene dichloride. The methylene dichloride extracts were combined, and the combined extracts washed three times with 50-75 ml portions of saturated aqueous sodium bicarbonate. The organic layer was then dried, and the solvent removed in vacuo to yield 2.96 g of a pale yellow oil comprising 4-di-n-propylaminocyclohexanone formed in the above reaction. The compound showed the expected carbonyl peak at 5.85μ in the infrared. The infrared also showed no hydroxyl absorption.

A solution was prepared by dissolving 1 g of 4-di-n-propylaminocyclohexanone in 7.5 ml of glacial acetic acid. 1.2 ml of 31% hydrogen bromide in glacial acetic acid was added followed by the dropwise addition of 0.195 ml of bromine. The reaction mixture was stirred under nitrogen for about ½ hour. The volatile constituents were then removed in vacuo to leave a brown viscous oil comprising 2-bromo-4-di-n-propylaminocyclohexanone formed in the above reaction. 420 mg of thiourea were added to this residue followed by 10 ml of anhydrous ethanol. The mixture was stirred to give a brown solution. The solution was heated to reflux temperature under a nitrogen atmosphere for about 2 hours. The reaction mixture was stirred overnight at ambient temperature, then filtered. The recovered solid, comprising (±)-2-amino-5-di-n-propylamino-4,5,6,7-benzothiazole dihydrobromide formed in the above reaction weighed 1.05 g. The dihydrobromide salt was recrystallized from a methanol/ether solvent mixture to yield 589 mg of purified dihydrobromide salt.

Analysis calculated: C, 37.60; H, 6.07; N, 10.12;
Found: C, 37.63; H, 5.72; N, 10.00.

Mass spectrum, molecular ion (-2HBr) at 253.

The corresponding 2-aminotetrahydrobenzoxazole can be prepared in a similar fashion by substituting urea for thiourea in the above reaction.

Example 2

Preparation of (±)-2-Amino-6-dimethylamino-5,6,7,8-tetrahydrobenzothiazole

Ten grams of 1,4-cyclohexanedione-mono-2,2-dimethyltrimethylene ketal were mixed with 50 g of anhydrous dimethylamine in benzene solution at about 5°C. 4.75 g of $TiCl_4$ in benzene was added thereto in dropwise fashion. A precipitate formed. The reaction mixture was allowed to warm to room temperature at which temperature it was stirred for about 1 hour. A solid which formed was separated by filtration. Evaporation of the solvent from the filtrate yielded about 9.88 g of an oil which was hydrogenated at 60 psi at room temperature overnight in anhydrous ethanol with 0.5 g of Pd/C as the catalyst. The catalyst was removed by

filtration. Evaporation of the filtrate in vacuo gave 8.82 g of an oil comprising 4-dimethylaminocyclohexane 2,2-dimethyltrimethylene ketal formed in the above reaction. TLC showed one spot ($SiO_2$/EtOAc).

Proton NMR ($CDCl_3$) δ 1.0 (s, 6H); 1.2-1.9 (m, 6H); 2.0-2.5 (m, 9H); 3.4 (s, 4H).

The ketal group was removed as follows: 0.37 g of the above ketal were dissolved in 90% aqueous formic acid and the resulting solution heated at 85-90°C for about 1 hour. The formic acid was removed by evaporation in vacuo. The resulting residue was dissolved in $CH_2Cl_2$ and the organic solution extracted twice with approximately equal volumes of 1N hydrochloric acid. The acidic extracts were combined. Evaporation to dryness gave 0.2 g of 4-dimethylaminocyclohexanone hydrochloride [one spot by TLC ($SiO_2$/MeOH/AcOH)]. Two repeat runs with 1.58 g and 3.19 g of starting ketal gave 1.26 g and 2.35 g of amino ketone, respectively.

Proton NMR ($CDCl_3$/$DMSOd_6$) δ 1.80-2.30 (m, 2H); 2.35-2.68 (m, 6H); 2.85 (d, 6H); 3.5-3.9 (m, 1H).

Mass spectrum: molecular ion at 141

Infrared spectrum: peak at 1725 $cm^{-1}$.

Following the procedure of Example 1, 1.26 g of 4-dimethylaminocyclohexanone was brominated alpha to the carbonyl to yield 2-bromo-4-dimethylaminocyclohexanone, which derivative was reacted without further purification with thiourea. The product of this reaction, (±)-2-amino-6-dimethylamino-5,6,7,8-tetra-

hydrobenzothiazole, was isolated as the dihydrobromide salt.

Mass spectrum: molecular ion at 197

Infrared spectrum: peak at 1626.11cm$^{-1}$

Analysis: Calcd.: C, 30.10; H, 4.77; N, 11.70; Br, 44.50;

Found: C, 29.95; H, 4.51; N, 11.72; Br, 44.58.

As previously stated, the compounds of this invention (wherein neither $R^3$ nor $R^4$ is H) are receptor agonists; ie, they are capable of increasing the quantities of various neurohormones--dopamine and norepinephrine in particular--available for interaction with specific receptors. For example, the compound according to XX wherein $R^1$ and $R^2$ are H and $R^3$ and $R^4$ are both n-propyl, is a specific dopamine D-2 agonist. One of such dopamine agonist activities is the inhibition of prolactin secretion, as demonstrated according to the following protocol.

Adult male rats of the Sprague-Dawley strain weighing about 200 g were housed in an air-conditioned room with controlled lighting (lights on 6 a.m. - 8 p.m.) and fed lab chow and water ad libitum. Each rat received an intraperitoneal injection of 2.0 mg of reserpine in aqueous suspension 18 hours before administration of the test drug. The purpose of the reserpine was to keep the rat prolactin levels uniformly elevated. The compound was dissolved in 10 percent ethanol, and injected intraperitoneally to groups of ten

rats at preselected dose levels. A control group of 10 intact males received an equivalent amount of 10 percent ethanol. One hour after treatment, all rats were killed by decapitation, and 150 μl aliquots of serum were assayed for prolactin.

The difference between the prolactin levels of the treated rats and the control rats, divided by the prolactin level of the control rats gives the percent inhibition of prolactin secretion attributable to the given dose. Inhibition percentages are given in Table 1 below for (±)-2-amino-6-di-n-propylamino-5,6,7,8-tetrahydrobenzothiazole dihydrobromide. In the table, column 1 gives the dose and column 2, the percent prolactin inhibition at the specified dose level.

Table 1

| Dose | % Inhibition |
|---|---|
| 50 mcg/kg | 30 |

Dopamine agonists are also known to affect turning behavior in 6-hydroxydopamine-lesioned rats in a test procedure designed to uncover compounds useful for the treatment of Parkinsonism. In this test, nigroneostriatal-lesioned rats are employed, as prepared by the procedure of Ungerstedt and Arbuthnott, Brain Res, 24, 485 (1970). A compound having dopamine agonist activity causes the rats to turn in circles contralateral to the side of the lesion. After a latency period,

which varies from compound to compound, the number of turns is counted over a 15-minute period.

Results obtained from such testing for (±)-2-amino-6-di-n-propylamino-5,6,7,8-tetrahydrobenzothiazole dihydrobromide are set forth in Table 2 below. In the table, column 1 gives the dose and column 2 the average number of turns observed in first 15 minutes after the end of the latency period.

Table 2

| Dose | Average number of Turns |
|---|---|
| 1000 mcg/kg | 51 |

Compounds according to XX, particularly those wherein both $R^3$ and $R^4$ are methyl, reduce the blood pressure of anesthetized spontaneously hypertensive rats, as illustrated by the following experiment:

Adult male spontaneously hypertensive rats (SHR) (Taconic Farms, Germantown, New York), weighing approximately 300 g. were anesthetized with pentobarbital sodium (60 mg./kg., i.p.). The trachea was cannulated and the SHR respired room air. Pulsatile arterial blood pressure was measured from a cannulated carotid artery using a Statham transducer (P23 ID). Mean arterial blood pressure was calculated as diastolic blood pressure plus 1/3 pulse pressure. Cardiac rate was monitored by a cardiotachometer which was triggered by the systolic pressure pulse. Drug solutions were administered i.v. through a catheter placed in a femoral vein.

Arterial blood pressure and cardiac rate were recorded on a multichannel oscillograph (Beckman, Model R511A). Fifteen minutes were allowed to elapse following surgery for equilibration of the preparation.

Table 3 which follows gives the results of this test using (±)-2-amino-6-di-n-propyl-5,6,7,8-tetrahydrobenzothiazole dihydrobromide. In Table 3, column 1 gives the dose level, column 2, the change in mean arterial blood pressure.

Table 3

| Dose in mcg/kg | Decrease in Mean Arterial Blood Pressure |
|---|---|
| 1 | -16.1 ± 2.4 |
| 10 | -21.0 ± 2.0 |
| 100 | -34.1 ± 3.2 |
| 1000 | +13.5 ± 4.4 |

In another test for hypotensive action conscious SHR, cannulated in the femoral artery and vein, were used. The drug was administered i.v. (±)-2-Amino-6-dimethylamino-5,6,7,8-tetrahydrobenzothiazole was tested in this system as the dihydrobromide salt at a 1 mg/kg dose. Table 4 gives the results of this test. In the table, column 1 gives time in minutes after injection and columns 2 and 3 the mean percent decrease in arterial pressure and heart rate respectively for that time interval.

Table 4

| Time after injection in minutes | PERCENT CHANGE FROM CONTROL Blood Pressure | Heart Rate |
|---|---|---|
| 5 | -3.6 | -12.3 |
| 15 | -17.1 | -17.7 |
| 30 | -18.6 | -15.5 |
| 45 | -16.9 | -8.3 |
| 60 | -17.2 | -5.2 |
| 90 | -16.9 | -0.7 |
| 120 | -15.8 | -4.1 |
| 180 | -14.7 | -4.1 |

The compounds of this invention are administered for therapeutic purposes in a variety of formulations including hard gelatin capsules prepared by mixing the drug with these ingredients: dried starch, dried magnesium stearate. The drug plus ingredients are mixed and filled into hard gelatin capsules each containing an effective dose of the drug.

Tablets are prepared by blending the drug Cellulose, microcrystalline; fumed Silicon dioxide, and Stearic acid. The components are blended and compressed to form tablets.

Suspensions are made by passing the drug through a No. 45 mesh U.S. sieve and mixing the sieved drug with sodium carboxymethylcellulose and syrup to form a smooth paste. A preservative, flavor and color are diluted with water and added with stirring. Sufficient water is then added to produce the required volume.

CLAIMS

1. A compound of Formula XX:

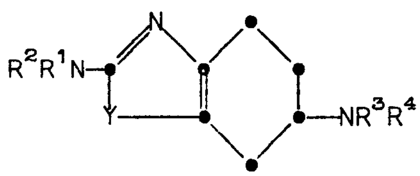

XX

wherein $R^1$ and $R^2$ are individually H, methyl, ethyl, or n-propyl and wherein $R^3$ and $R^4$ are individually H, methyl, ethyl, n-propyl or allyl; Y is S or O; or a pharmaceutically-acceptable, acid addition salt thereof.

2. A compound according to Claim 1 wherein neither $R^3$ nor $R^4$ is H.

3. A compound according to Claim 1 or 2 wherein both $R^1$ and $R^2$ are H.

4. A compound according to Claim 1 or 2 wherein $R^1$ and $R^2$ are each either H or methyl.

5. A dihydrobromide salt of a compound according to Claim 2.

6. A compound according to Claims 1 to 4 in which both $R^3$ and $R^4$ are n-propyl.

7. A compound according to Claim 1 wherein Y is S.

8. (±)-2-Amino-6-di-n-propylamino-5,6,7,8-tetrahydrobenzothiazole.

9. (±)-2-Amino-6-dimethylamino-5,6,7,8-tetrahydrobenzothiazole.

10. A process for preparing a compound of Formula XX, as claimed in any one of claims 1 to 9, which comprises:
reacting a compound of Formula A

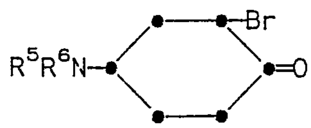

A

with a compound of Formula B,

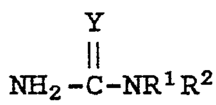

B ,

optionally dealkylating the 6 amino group and realkylating to yield different $R^5$ and $R^6$ substituted amines, and/or optionally salifying.

11. A compound of Formula XX as claimed in any one of claims 1 to 9 for use as a dopamine receptor agonist.

12. A pharmaceutical formulation comprising as an active ingredient a compound of Formula XX or a pharmaceutically acceptable salt thereof, as claimed in any one of claims 1 to 9, associated with one or more pharmaceutically-acceptable carriers therefor.

# CLAIMS

1. A process for preparing a compound of Formula XX:

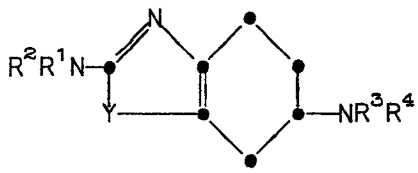

XX

wherein $R^1$ and $R^2$ are individually H, methyl, ethyl, or n-propyl and wherein $R^3$ and $R^4$ are individually H, methyl, ethyl, n-propyl or allyl; Y is S or O; or a pharmaceutically-acceptable, acid addition salt thereof, which comprises:
reacting a compound of Formula A

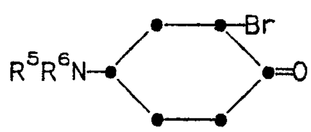

A

with a compound of Formula B,

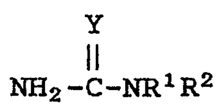

B ,

optionally dealkylating the 6 amino group and realkylating to yield different $R^5$ and $R^6$ substituted amines, and/or optionally salifying.

2. A process according to Claim 1 wherein neither $R^3$ nor $R^4$ is H.

3. A process according to Claim 1 or 2 wherein both $R^1$ and $R^2$ are H.

4. A process according to Claim 1 or 2 wherein $R^1$ and $R^2$ are each either H or methyl.

5. A process according to Claim 1, for preparing a dihydrobromide salt wherein neither $R^3$ nor $R^4$ is H.

6. A process according to Claims 1 to 4 in which both $R^3$ and $R^4$ are n-propyl.

7. A process according to Claim 1 wherein Y is S.

8. A process according to Claim 1 for preparing (±)-2-Amino-6-di-n-propylamino-5,6,7,8-tetrahydrobenzothiazole.

9. A process according to Claim 1 for preparing (±)-2-Amino-6-dimethylamino-5,6,7,8-tetrahydrobenzothiazole.

European Patent Office

# EUROPEAN SEARCH REPORT

0207696

Application number

EP 86 30 4754

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
| --- | --- | --- | --- |
| A | DE-A-2 136 233 (RIEDEL-DE HAEN AG)<br>* page 14, lines 10-23 *<br>--- | 1,10 | C 07 D 277/82<br>C 07 D 263/58<br>A 61 K 31/425<br>A 61 K 31/42 |
| A | FR-A-1 566 157 (LABORATOIRES DAUSSE/B.M.C.)<br>* claims 1, 2; example 1 *<br>----- | 1,10 | |
| | | | **TECHNICAL FIELDS SEARCHED (Int Cl.4)** |
| | | | C 07 D 263/00<br>C 07 D 277/00 |

The present search report has been drawn up for all claims

| Place of search | Date of completion of the search | Examiner |
| --- | --- | --- |
| BERLIN | 18-09-1986 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82